# EUROPÄISCHE PATENTANMELDUNG

(11) **EP 3 738 615 A1**
(43) Veröffentlichungstag der Anmeldung: **18.11.2020**
(21) Anmeldenummer: 20169781.0
(22) Anmeldetag: 16.04.2020
(51) Int. Cl.: A61L 2/08, A61L 2/10, A61L 2/18, A61L 2/20, A61L 2/22, C02F 1/32, A61L 9/14, A61L 9/18

(54) **VERFAHREN ZUR REDUZIERUNG DER ANZAHL VON KEIMEN**

(30) Priorität: 16.04.2019 EP 19169430
(71) Anmelder: HUBL GmbH Edelstahltechnik, 71665 Vaihingen/Enz (DE)
(72) Erfinder: KIEFER, Rainer, 71254 Ditzingen (DE)
(74) Vertreter: Jeck, Anton

(57) **Zusammenfassung**

Die vorliegende Erfindung betrifft ein Verfahren zur Reduzierung der Anzahl von Keimen in einem gasförmigen Medium, insbesondere in der Luft, bei welchem eine Reinigungssubstanz als Aerosol oder als Rauch bereitgestellt wird, das Aerosol oder der Rauch in dem gasförmigen Medium zum Bilden eines Gemisches verteilt wird, das Gemisch mit einer elektromagnetischen Strahlung mit mindestens einer definierten Wellenlänge gleichmäßig bestrahlt wird und das Aerosol oder der Rauch im Zuge der Bestrahlung des gasförmigen Gemisches reduziert.

## Beschreibung

Die Erfindung betrifft ein Verfahren zur Reduzierung der Anzahl von Keimen in oder auf einem Medium gemäß Anspruch 1 sowie eine Vorrichtung zur Reduzierung von Keimen in oder auf einem Medium gemäß Anspruch 9.

Dem Fachmann sind eine Vorrichtung sowie ein Verfahren bekannt, bei welchen ein zu desinfizierendes Objekt durch eine Vorreinigung geschleust wird, wobei in der Vorreinigungsvorrichtung das zu entkeimende Objekt gereinigt und anschließend mit einem Reinigungsfluid besprüht wird. Durch Bestrahlung wird eine Desinfizierung der Oberfläche erreicht.

Der vorliegenden Erfindung liegt daher die **Aufgabe** zugrunde, ein Verfahren zur effizienten Reduzierung von Keimen in gasförmigen Medien zu entwickeln.

Die Aufgabe wird zum einen durch ein Verfahren gemäß dem Verfahrensanspruch 1 sowie durch eine Vorrichtung gemäß dem Vorrichtungsanspruch 9 gelöst.

Vorteilhafte Ausführungsformen der vorliegenden Erfindung sind in den Ansprüchen angegeben.

Das erfindungsgemäße Verfahren ist dadurch gekennzeichnet, dass eine Reinigungssubstanz als Gas, Aerosol oder als Rauch bereitgestellt wird, das Gas, das Aerosol oder der Rauch in dem gasförmigen Medium zum Bilden eines Gemisches verteilt wird, das Gemisch mit einer elektromagnetischen Strahlung mit mindestens einer definierten Wellenlänge gleichmäßig bestrahlt wird und das Aerosol oder der Rauch im Zuge der Bestrahlung des gasförmigen Gemisches reduziert.

Die Reinigungssubstanz kann grundsätzlich als ein Gas, ein Aerosol ein Rauch oder ein Pulver, vorliegen. Die Reinigungssubstanz kann auch als Reinigungsmittel bezeichnet werden, muss nach einer Ausführungsform den oder die maßgeblichen Wirkstoffe jedoch nicht in Reinform, enthalten. Es können insbesondere Hilfsstoffe vorgesehen sein, welche es erleichtern die Reinigungssubstanz für eine Gasdesinfektion bereitzustellen. Die Hilfsstoffe können auch als Trägersubstanzen für den Phasenüberganz bezeichnet werden.

Die kann beispielsweise Ethylenglykol oder eine, vorzugsweise bei niederer Verbrennungstemperatur, rauchbildende Substanz sein.

Ein Hilfsstoff für den Übergang der Reinigungssubstanz in den erfindungsgemäß verwendeten Zustand kann beispielsweise zur Stoffgruppe der Cellulosenitate (Schießbaumwolle) gehören oder ein Rauchbildner sein.

Beispielswiese Ethylenglycol kann beispielsweise in bekannter Weise als Aerosol bereitgestellt werden, in welchem die Reinigungssubstanz gelöst oder als Dispersion vorliegen kann.

Grundsätzlich ist auch eine Zerstäubung mit oder ohne Hilfsstoff denkbar. Dazu kann die Reinigungssubstanz beispielsweise Suspendier, geschmolzen oder aufgelöst werden.

Grundsätzlich kann die Reinigungssubstanz auch in Reinform in erfindungsgemäßer weise als Gas, Rauch oder Aerosol der Gasphase, insbesondere der zu reinigenden Luft, zuführbar sein.

Ein Grundgedanke der vorliegenden Erfindung besteht darin, ein mit Keimen belastetes gasförmiges Medium, mit einer Reinigungssubstanz, zu versetzen, das vorzugsweise gleichmäßig in dem gasförmigen Medium vorsehbar, vorzugsweise lösbar (Gas in Gas) oder suspendierbar ist, um dieses Gasphasengemisch einer Strahlungsquelle auszusetzen, insbesondere einer Strahlungsquelle für elektromagnetische Strahlung, vorzugsweise ein Leuchtmittel für sichtbares Licht. Hierdurch wird eine Verringerung der Anzahl der Keime, in dem Medium erreicht. Dabei kommt es vorzugsweise zu einer Wechselwirkung zwischen der Strahlung und der Substanz, wodurch diese aktiviert wird und die aktivierte Substanz zur Entkeimung des Mediums beiträgt oder diese bewirkt. Somit kann eine doppelte Wirkung der Strahlung erreicht werden, nämlich eine direkte Einwirkung der Strahlung auf die Keime und eine daraus resultierende Reduzierung der Anzahl der Keime sowie eine Aktivierung der Substanz in dem Medium und eine anschließende Reduzierung der Anzahl der Keime mittels die aktivierte Substanz.

Erfindungsgemäß ist unter einer reduzierten Anzahl von Keimen die Anzahl von pathogenen Keimen, wie beispielsweise Viren, Bakterien oder Pilze, in ihrer aktiven Form zu verstehen. Zwar können die Keime nach der erfindungsgemäßen Reduzierung ihrer Anzahl immer noch in dem Medium vorliegen. Ihre Wirkung auf biologisches Material, insbesondere auf Menschen oder Tiere, kann jedoch dennoch eingeschränkt oder aufgehoben sein. Somit wird erfindungsgemäß die Anzahl der Keime in dem Medium betrachtet, welche "lebend" bzw. biologisch aktiv vorliegt. Es handelt sich demnach um einen "Abtötungsprozess", bei welchem die Zahl der "toten" beziehungsweise inaktiven Keime bei der erfindungsgemäß betrachteten reduzierten Anzahl keine Berücksichtigung findet.

Grundsätzlich kann die Substanz, welche nachfolgend als Reinigungssubstanz bezeichnet wird, sowohl vor als auch nach der Verkeimung dem gasförmigen Medium beigemengt werden. Demnach entsteht die entkeimende Wirkung der Reinigungssubstanz besonders durch seine strahlungsbedingte Aktivierung. Nach einer bevorzugten Ausführungsform kann das Reinigungsfluid unmittelbar vor (oder während) der Bestrahlung dem Medium beigemengt werden.

Die Wechselwirkung zwischen der Strahlung und der Reinigungssubstanz, welche zu einer Aktivierung der Reinigungssubstanz führt, kann insbesondere eine selektive Wechselwirkung sein.

Die Reinigungssubstanz kann beispielsweise 10H-Benzo[g]pteridin-2,4-dion-Derivate sein. Vorzugsweise sind einzelne oder eine Kombination von einzelnen Wellenlängen des elektromagnetischen Strahlungsspektrums geeignet, die Aktivierung der Reinigungssubstanz zu bewirken. Hierbei kann es sich um die Strahlung mit einer definierten Wellenlänge oder um eine Strahlung mit einer Kombination von Wellenlängen handeln. Grundsätzlich kann es mehr als eine Wellenlänge geben, welche jeweils einzelnen oder in Kombination mit weiteren Wellenlängen geeignet ist, das Reinigungsfluid zu aktivieren. Hierbei kann erfindungsgemäß eine definierte Wellenlänge sowohl eine diskrete Wellenlänge X nm ± 0 nm als auch ein Wellenlängenbereich sein (X nm ± Y nm; wobei Y zwischen 0,1 und 50 nm ist), in welchem eine Aktivierung des Reinigungsfluids möglich ist. X kann insbesondere 405 nm sein. Vorzugsweise handelt es sich um Licht, also eine elektromagnetische Strahlung im sichtbaren Bereich, insbesondere im Bereich von 380 bis 780 nm.

## Patentansprüche

1. Verfahren zur Reduzierung der Anzahl von Keimen in einem gasförmigen Medium, insbesondere in der Luft, bei welchem
- eine Reinigungssubstanz als Aerosol oder als Rauch bereitgestellt wird,
- das Aerosol oder der Rauch in dem gasförmigen Medium zum Bilden eines Gemisches verteilt wird,
- das Gemisch mit einer elektromagnetischen Strahlung mit mindestens einer definierten Wellenlänge gleichmäßig bestrahlt wird und
- das Aerosol oder der Rauch im Zuge der Bestrahlung des gasförmigen Gemisches reduziert.
